# EUROPÄISCHE PATENTSCHRIFT

(11) **EP 3 768 196 B1**
(45) Veröffentlichungstag und Bekanntmachung des Hinweises auf die Patenterteilung: **16.03.2022**
(21) Anmeldenummer: 19722780.4
(22) Anmeldetag: 20.03.2019
(51) Int. Cl.: A61F 2/32, A61N 1/05, A61N 1/36, A61F 2/30, A61F 2/36, A61F 2/48

(54) **PROTHESENSYSTEM**
PROSTHESIS SYSTEM
SYSTÈME DE PROTHÈSE

(30) Priorität: 23.03.2018 DE 102018106945
(43) Veröffentlichungstag der Anmeldung: 27.01.2021
(73) Patentinhaber: FRAUNHOFER-GESELLSCHAFT zur Förderung der angewandten Forschung e.V., 80686 München (DE)
(72) Erfinder: LAUSCH, Holger, 04299 Leipzig (DE); FABIAN, Claire, 04317 Leipzig (DE)
(74) Vertreter: Gleim Petri Oehmke Patent- und Rechtsanwaltspartnerschaft mbB
(86) Internationale Anmeldenummer: PCT/DE2019/100262
(87) Internationale Veröffentlichungsnummer: WO 2019/179578

(56) Entgegenhaltungen:
- EP-A1- 3 231 360
- DE-U1-202009 004 348
- US-A1- 2013 079 673
- US-A1- 2014 303 539

## Beschreibung

Die Erfindung betrifft ein Prothesensystem zur vollständigen oder teilweisen Implantation in einen menschlichen Körper.

Bei endoprothetischen Implantationen im Knie-, Hüft- und Schultergelenkbereich sowie bei Totalrevisionen, z. B. des kompletten Oberschenkelknochens, gehen in nicht wenigen Fällen unweigerlich die vorherigen neuronalen Anbindungen an das Knochen- und Muskelgewebe in Gestalt der Propriozeptoren verloren.

Als Propriozeptoren bezeichnet man Mechanorezeptoren, welche die Wahrnehmung der Lage, Kraft, Stellung und Bewegung des Körpers im Raum gewährleisten. Durch sie gelangen Informationen über Muskelspannung, Muskellänge, Gelenkstellung und Bewegung zum Kleinhirn und zum Cortex, wo diese verarbeitet werden. Lokalisiert sind die Propriozeptoren in Gelenken, partizipierender Muskulatur, Sehnen, Bändern, Bindegewebe und der über den entsprechenden Gelenken liegenden Haut. So besitzt das vordere Kreuzband propriozeptive Nervenfasern, über die dem Zentralnervensystem Informationen über die Bewegungsabläufe des Kniegelenks zugeleitet werden. Mit einer Störung der Propriozeption geht eine Minderung des Koordinationsvermögens einher. Die Folge sind einerseits vorzeitiger Verschleiß des Implantats und/oder des restständigen, benachbarten knöchernen Gewebes durch nicht wahrnehmbare Fehl- und Überbelastung, sowie andererseits zusätzliche Komplikationen durch Stürze und Brüche (vor allem des schlecht heilenden Oberschenkelhalses) aufgrund des geminderten Koordinationsvermögens besonders bei älteren, unter Osteoporose leidenden Patienten.

Das Prinzip der Neurostimulation an sich ist seit Jahrzehnten Gegenstand der Forschung und wird bereits in vielen klinischen Bereichen angewendet. Bekannt sind verschiedenste Formen der Neurostimulation über externe Eingriffe, neuronale Schrittmacher, etc. Diese werden vor allem im Bereich des zentralen Nervensystems eingesetzt, um pathologischen Funktionsverlusten vorzubeugen oder bei eingeschränkten oder verloren gegangenen Funktionen diese wiederherzustellen, zu verbessern und/oder zu ersetzen. Dies basiert u. a. auf der Verbesserung der Neurogenese und/oder dem Erhalt der neuronalen Verknüpfungen durch Neurostimulation und/oder Modulation vorhandener neuronaler Netzwerke.

Im peripheren Nervensystem (Rückenmark) werden zur Therapie von chronischen Schmerzen Neuroimplantate eingesetzt. Diese Systeme umfassen temporäre Anbindung an Elektroden mit einer offenen Verbindung oder komplett implantierbare, batteriebetriebene dauerhafte Systeme, wobei auch hier eine Erneuerung der Batterie nach einigen Jahren notwendig ist. Die Stimulation erfolgt dabei unabhängig von intrinsischen, körpereigenen Signalen.

Mit der DE 20 2009 004 348 U1 wird eine Endoprothese mit einem integrierten Lockerungssensor beschrieben. Gemäß der offenbarten Anordnung umfasst das Lockerungsmesssystem einen an einer Feder aufgehängten bzw. fixierten, freibeweglichen ferromagnetischen Massenschwinger, der eine anliegende Membran zum Schwingen anregen kann, Desweiteren kann auch ein Piezoelement mit geschalteter Spule und Kapazität direkt an eine anliegende Membran angekoppelt werden, welche wiederum über die Endoprothese in Schwingung versetzt werden kann. Die sogenannten Lockerungssensoren lassen sich dabei einzeln ansprechen, sofern deren Eigenfrequenzen in ausreichendem Maße voneinander abweicht. Diese Systeme sind nur von außen (ex vivo) über Spulen anregbar und oder auslesbar. Ein biomechanisch-neurobiologischer Schwingkreis über eine natürlich, nicht normierte und wechselnde Endoprothesenbelastung z. B. beim Laufen, Springen oder Treppen gehen etc. ist nicht vorgesehen.

Die US 2013/0079673 A1 offenbart einen orthopädischen Messeinsatz mit sterilem Gehäuse, der Teil einer Gelenkprothese oder eines entsprechend präparierten Gelenkknochens sein kann. Mit dem Messeinsatz können verschiedenste Bewegungs- und Belastungsparameter an der Gelenkprothese erfasst werden. Im Gehäuse ist eine elektronische Schaltung mit entsprechenden Sensoren und einer Energiequelle aufgenommen. Je nach verwendeten Sensoren könne Kräfte, Wege, Beschleunigungen, Viskositäten, pH-Werte oder Temperaturen erfasst werden. Die ermittelten Daten können mittels Antenne und Übertragungselektronik extern erfasst und aufgezeichnet werden. Die Energiequelle kann drahtlos, über elektromagnetische Kopplung wieder aufgeladen werden.

In der europäischen Patentanmeldung EP 3 231 360 A1 wird eine Prothesenkomponente, bestehend aus einem Sensor und weiteren elektronischen Komponenten zur Energiespeicherung, Energieübertragung, selektiver Signalübermittling und Visualisierung beschrieben. Mit diesem System soll es möglich sein, die spezifische Bewegungsweise von Prothesenbestandteilen zueinander (z. B. in einem Knieimplantat) aufzuzeichnen. Diese Daten sollen zu ihrer Intepretation nach ausserhalb des Körpers übermittelt werden.

Der Erfindung liegt die Aufgabe zugrunde, eine Möglichkeit anzugeben, wodurch fehlendes oder fehlgeleitetes neuronales Feedback nach einer endoprothetischen Implantation neu und komplementär erzeugt werden kann.

Erfindungsgemäß wird die Aufgabe gelöst durch ein Prothesensystem, bestehend aus einer Prothese und einer von dieser Prothese räumlich getrennten, als bioelektrischer Wandler wirkenden elektromagnetischen Wandlerspule mit Spulenenden, wobei an oder in der Prothese ein primärer elektromechanischer Wandler mit einem direkt gekoppelten primären elektromagnetischen Wandler zur Wandlung von mechanischer Energie in elektromagnetische Wellen, hervorgerufen durch Bewegungs-, Kraft-, Druck- und Belastungswechsel wirkend an oder in der Prothese, angeordnet ist und die davon räumlich getrennten Spulenenden der die elektromagnetischen Wellen in elektrische Signale wandelnden sekundären elektromagnetischen Wandlerspule als Elektroden in einem keramischen oder kunststoffbasierten Formkörper, der elektrisch nicht leitend und vorzugsweise offenporig ist, angeordnet sind.

Der primäre elektromechanische Wandler und der primäre elektromagnetische Wandler können dabei in einer Baukomponente vereinigt oder räumlich getrennt angeordnet sein.

Eine bevorzugte Ausführungsform besteht darin, dass der primäre elektromechanische Wandler in der Prothese ein Piezoelement ist, wobei das auf Bewegungs- und Belastungswechsel reagierende Piezoelement elektrische Spannungssignale erzeugt, welche der angekoppelte primäre elektromagnetische Wandler in ein elektromagnetisches Wechselfeld wandelt.

Vorteilhaft ist es, wenn die sekundäre elektromagnetische Wandlerspule keramisch und elektrisch nicht leitend eingehaust ist.

Um nach der erfolgten Implantation ein selbständiges Hineinwachsen von neuronalem Gewebe in den Formkörper zu stimulieren, ist es von Vorteil, wenn die Poren des Formkörpers mit aktiven oder elektrisch aktivierbaren Signalstoffen und/oder einer biologischen Matrix versehen werden. Das Hineinwachsen von neuronalem Gewebe in den Formkörper schafft so eine dauerhafte Signalschnittstelle zwischen neuronalem Gewebe und der über die Spulenenden verbundenen elektromagnetischen Wandlerspule. Der offenporige Formkörper kann dabei räumlich getrennt von der sekundären elektromagnetischen Wandlerspule oder als Bestandteil des Housing der sekundären elektromagnetischen Wandlerspule angeordnet sein.

Weiterhin kann es von Vorteil sein, wenn die sekundäre elektromagnetische Wandlerspule als Trigger mit einem Verstärkermodul gekoppelt wird.

Weitere vorteilhafte Ausführungen ergeben sich dadurch, dass die sekundäre elektromagnetische Wandlerspule einen integrierten Wechsel-/Gleichspannungsrichter aufweist oder die elektromagnetische Wandlerspule mit einem Energiespeicher und einem Signalgenerator, der aus einem unregelmäßigen Biofeedbacksignal ein immer gleichartiges Signal als Biofeedbacksignal generiert, welches nur in der Frequenz oder Amplitude differiert, in Verbindung steht.

Nach erfolgter Implantation der Prothese ist die keramisch eingehauste sekundäre elektromagnetische Wandlerspule örtlich in die Reichweite der durch den primären elektromagnetischen Wandler erzeugten elektromagnetischen Wellen zu platzieren. Damit ein erfolgreiches Biofeedbacksystem mit dem erfindungsgemäßen Prothesensystem aufgebaut werden kann, sollte der Formkörper in unmittelbarer Nähe zu einem Nerv positioniert werden, damit möglichst schnell die dauerhafte Signalschnittstelle zwischen dem neuronalen Gewebe und der sekundären elektromagnetischen Wandlerspule entstehen kann.

Ein besonderer Vorteil des erfindungsgemäßen Prothesensystems besteht darin, dass eine direkte bioelektrische, energieautarke und lebenslange Verbindung zwischen dem Implantatgewebebereich und dem neuronalen System entsteht. Das vegetative System kann somit mittels des erfindungsgemäßen Prothesensystems Überlastungs- und Fehlverhalten direkt erfassen und durch Verhaltenskorrektur gegensteuern.

Unter einer Prothese soll sowohl jegliches Implantat, ob geschlossenes (Endoprothese) oder offenes (Zahnimplantat oder Epithesen) Implantat, als auch eine Exoprothese verstanden sein. Bei der Exoprothese sind der primäre elektromechanische und der elektromagnetische Wandler an der Grenzfläche zum Gliedmaßenstumpf angeordnet. Die neuronale Schnittstelle ist dann im Stumpf implantiert. Ziel ist dann neuronales Tiefenempfinden für eine bessere Durchblutung zu erreichen.

Nachfolgend soll die Erfindung anhand von Ausführungsbeispielen näher erläutert werden. Die Zeichnungen zeigen in
- Fig. 1: ein implantiertes erfindungsgemäßes Prothesensystem in Form eines Hüftgelenksimplantats,
- Fig. 2: ein implantiertes erfindungsgemäßes Prothesensystem in Form eines Unterschenkel-Knieimplantats,
- Fig. 3: eine Schnittdarstellung durch eine gekapselte sekundäre elektromagnetische Wandlerspule und
- Fig. 4: eine schematische Detaildarstellung A aus Fig. 1.

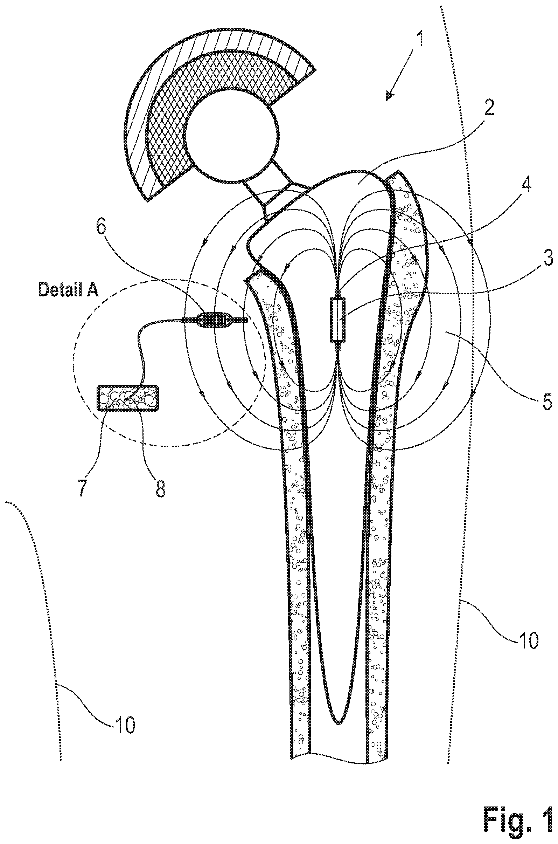

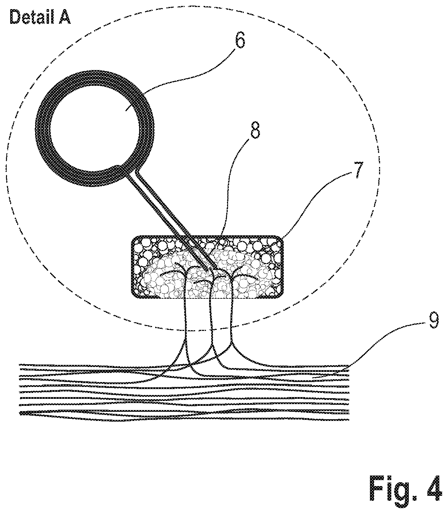

In Fig. 1 sind die wesentlichen Komponenten des erfindungsgemäßen Prothesensystems 1 dargestellt. Das Prothesensystem 1 besteht dabei aus der Endoprothese 2, hier als Hüftgelenksimplantat ausgeführt, dem primären elektromechanischen Wandler 3, dem primären elektromagnetischen Wandler 4, der die elektromagnetischen Wellen 5 erzeugen kann, und der keramisch eingehausten sekundären elektromagnetischen Wandlerspule 6 sowie dem Formkörper 7 als neuronale Schnittstelle. Der primäre elektromechanische Wandler 3 ist innerhalb der Endoprothese 2 derart angeordnet, dass er energieautark intrinsische Bewegungen bzw. Belastungsunterschiede/-wechsel in Spannungsänderungen umwandelt. Intrinsisch heißt in diesem Zusammenhang aus der Bewegung der Endoprothese 2 in sich und aus sich im Muskel-Skelett-Umfeld. Dies schließt insbesondere auch die Implantat-Gewebe-Kontaktzone ein, in und an der sich Belastungswechsel als Drücke und Kräfte auf das Implantat und damit auf den eingebetteten primären elektromechanischen Wandler 3 manifestieren. Der mit dem primären elektromechanischen Wandler 3 unmittelbar in Verbindung stehende primäre elektromagnetische Wandler 4 erzeugt durch diese Spannungsänderungen die in Fig. 1 schematisch dargestellten elektromagnetischen Wellen 5. Die im Wirkungsbereich dieser elektromagnetischen Wellen 5 angeordnete sekundäre elektromagnetische Wandlerspule 6, die als sekundäre Induktionsspule wirkt, detektiert diese und wandelt sie wieder in elektrische Signale. Die sekundäre elektromagnetische Wandlerspule 6 befindet sich gemäß Fig. 1 außerhalb der Endoprothese 2, ist jedoch wie die Endoprothese 2 im menschlichen Körper orthogonal zu den primären elektromagnetischen Wellen 5 positioniert und implantiert. Die rückgewandelten elektrischen Signale der sekundären elektromagnetischen Wandlerspule 6 werden über die Spulenenden 8 in den porösen Formkörper 7 eingeleitet. Der Formkörper 7 sollte möglichst in der Nähe eines Nervs 9 implantiert werden (siehe Fig. 4). Die Poren des Formkörpers 7 kann man mit einem biochemischen, signaltransduktiven Stoff befüllen, der für sich neuroaktiv sein kann, aber auch einerseits über bioelektrische Impulse aktivierbar ist und andererseits so signaltransduktiv wirkt, dass vom nächstgelegenen Nerv 9 neuronale Zellen und Gewebe in Richtung des Formkörpers 6 wachsen und sich so induktiv eine neuronale, bioelektrische Schnittstelle bildet. Dies kann auch allein oder in Kombination mit Scaffolds/Matrixproteinen (z. B. biokompatiblen Polymeren, Kollagenfasern) zur Verbesserung der Gewebegeneration erfolgen. Zusätzlich kann die Formgebung des Scaffolds verletzten Neuronen als "Leitbahnen" bei der Regeneration dienen und zusätzlich mit Zellen besiedelt werden, welche die neuronale bzw. Gewebegeneration unterstützen (z. B. Schwannsche Zellen, mesenchymale Stammzellen). Die elektromagnetische Wandlerspule 6 sollte keramisch ummantelt sein, um an der Oberfläche entstehende Wirbelströme und Felder in der Nähe des Nervs 9 zu verhindern.

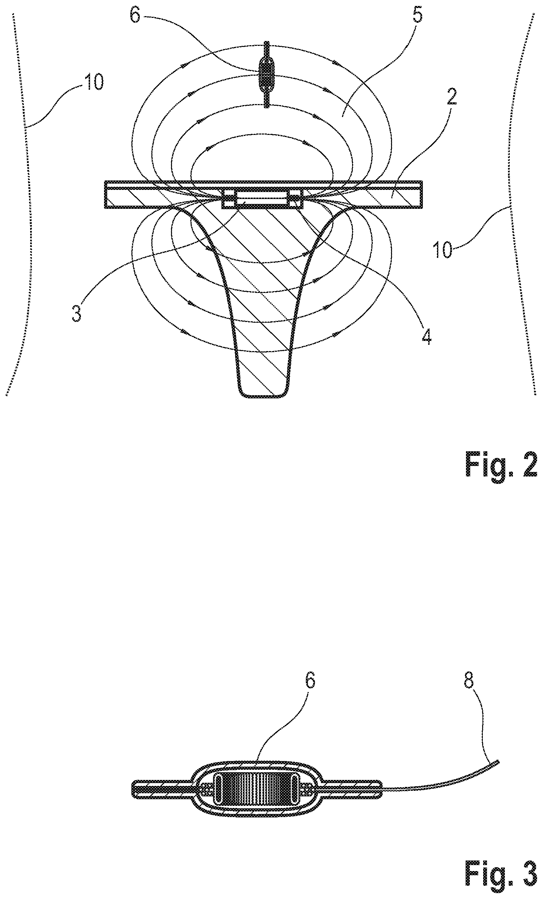

In Fig. 2 ist die Endoprothese 2 schematisch als Unterschenkel-Knieimplantat mit dem primären elektromechanischen Wandler 3 und dem primären elektromagnetischen Wandler 4, gemeinsam in einer Kavität liegend, dargestellt. Auch hier ist wieder die sekundäre elektromagnetische Wandlerspule 6 wie in Fig. 1 liegend orthogonal zu den elektromagnetischen Wellen 5 angeordnet.

Fig. 3 zeigt eine keramisch ummantelte sekundäre elektromagnetische Wandlerspule 6, wobei die keramischen Ober- und Unterhälften über eine Lot- oder Klebezone inert verbunden und biologisch dicht sind.

In Fig. 4 ist ein Nerv 9 schematisch durch die waagerechten Linien symbolisiert. In seiner unmittelbaren Nähe ist der poröse Formkörper 7 positioniert. Über die Spulenenden 8 ist der Formkörper 7 mit der sekundären elektromagnetischen Wandlerspule 6 verbunden. In den Formkörper 7 ist bereits gemäß Fig. 4 neuronales Gewebe hineingewachsen und bildet so eine dauerhafte neuronale Schnittstelle zwischen neuronalem Gewebe und sekundärer elektromagnetischer Wandlerspule 6. Zwischen Spulenenden 8 und elektromagnetischer Wandlerspule 6 kann eine zwischengeschaltete Wandlereinheit, die die Wechselspannung in eine Gleichspannung richtet, vorgesehen werden. Die Spulenenden 8 können für sich auch direkt oder über den porösen Formkörper indirekt als Gewebeelektroden angesehen werden.

Ferner kann die elektromagnetische Wandlerspule 6 mit einem Energiespeicher und einem Signalgenerator, der aus einem unregelmäßigen Biofeedbacksignal ein immer gleichartiges Signal als Biofeedbacksignal generiert, welches nur in der Frequenz oder Amplitude differieren kann, verschaltet sein.

### Bezugszeichenliste

- 1: Prothesensystem
- 2: Endoprothese
- 3: primärer elektromechanischer Wandler
- 4: primärer elektromagnetischer Wandler
- 5: elektromagnetische Wellen
- 6: sekundäre elektromagnetische Wandlerspule
- 7: Formkörper
- 8: Spulenenden
- 9: Nerv

## Patentansprüche

1. Prothesensystem (1), bestehend aus einer Prothese (2) und einer von der Prothese (2) räumlich getrennten, als bioelektrischer Wandler wirkenden eingehausten elektromagnetischen Wandlerspule (6) mit Spulenenden (8), wobei an oder in der Prothese (2) ein primärer elektromechanischer Wandler (3) mit einem direkt gekoppelten primären elektromagnetischen Wandler (4) zur Wandlung von mechanischer Energie in elektromagnetische Wellen (5), hervorgerufen durch Bewegungs-, Kraft-, Druck- und Belastungswechsel wirkend an oder in der Prothese (2), angeordnet ist, **dadurch gekennzeichnet, dass** die davon räumlich getrennten Spulenenden (8) der die elektromagnetischen Wellen (5) in elektrische Signale wandelnden sekundären elektromagnetischen Wandlerspule (6) als Elektroden in einem keramischen oder kunststoffbasierten Formkörper (7), der elektrisch nicht leitend ist, angeordnet sind.

2. Prothesensystem (1) nach Anspruch 1, **dadurch gekennzeichnet, dass** der primäre elektromechanische Wandler (3) ein Piezoelement ist, wobei das auf Bewegungs- und Belastungswechsel reagierende Piezoelement elektrische Spannungssignale erzeugt, welche der angekoppelte primäre elektromagnetische Wandler (4) in ein primäres elektromagnetisches Wechselfeld wandelt.

3. Prothesensystem (1) nach Anspruch 1, **dadurch gekennzeichnet, dass** der primäre elektromechanische Wandler (3) und der primäre elektromagnetische Wandler (4) in einer Baukomponente vereinigt oder räumlich getrennt angeordnet sind.

4. Prothesensystem (1) nach Anspruch 1, **dadurch gekennzeichnet, dass** der Formkörper (7) offenporig ausgebildet ist und die Poren des Formkörpers (7) mit aktiven oder elektrisch aktivierbaren Signalstoffen und/oder einer biologischen Matrix versehen sind, die nach Implantation des Formkörpers (7) ein selbständiges Hineinwachsen von neuronalem Gewebe in den Formkörper (7) stimulieren, sodass eine dauerhafte Signalschnittstelle zwischen neuronalem Gewebe und sekundärer elektromagnetischer Wandlerspule (6) ausgebildet ist.

5. Prothesensystem (1) nach Anspruch 1 bis 4, **dadurch gekennzeichnet, dass** der Formkörper (7) räumlich getrennt von der sekundären elektromagnetischen Wandlerspule (6) oder als Bestandteil der sekundären elektromagnetischen Wandlerspule (6) angeordnet ist.

6. Prothesensystem (1) nach Anspruch 1, **dadurch gekennzeichnet, dass** die sekundäre elektromagnetische Wandlerspule (6) als Trigger mit einem Verstärkermodul gekoppelt ist.

7. Prothesensystem (1) nach Anspruch 1, **dadurch gekennzeichnet, dass** die sekundäre elektromagnetische Wandlerspule (6) einen integrierten Wechsel-/Gleichspannungsrichter aufweist.

8. Prothesensystem (1) nach Anspruch 1, **dadurch gekennzeichnet, dass** die sekundäre elektromagnetische Wandlerspule (6) mit einem Energiespeicher und einem Signalgenerator, der aus einem unregelmäßigen Biofeedbacksignal ein immer gleichartiges Signal als Biofeedbacksignal generiert, welches nur in der Frequenz oder Amplitude differiert, in Verbindung stehend angeordnet ist.

9. Prothesensystem (1) nach einem der vorhergehenden Ansprüche zur Implantation in einen menschlichen Körper, **dadurch gekennzeichnet, dass** nach durchgeführter Implantation des Prothesensystems (1) die keramisch eingehauste sekundäre elektromagnetische Wandlerspule (6) in der Reichweite der durch den primären elektromagnetischen Wandler (4) erzeugten primären elektromagnetischen Wellen (5) und der Formkörper (7) in unmittelbarer Nähe zu einem Nerv (9) positioniert sind.

10. Prothesensystem (1) nach Anspruch 9, **dadurch gekennzeichnet, dass** die von der sekundären elektromagnetischen Wandlerspule (6) über die Spulenenden (8) gelieferten bioelektrischen Feedbacksignale als bioelektrisch-neuronales Feedback aus der Prothese (2) wieder für das vegetative System zur Verfügung stehen.

11. Prothesensystem (1) nach einem der vorhergehenden Ansprüche, **dadurch gekennzeichnet, dass** die elektromagnetische Wandlerspule (6) keramisch eingehaust ist.

12. Prothesensystem (1) nach einem der vorhergehenden Ansprüche, **dadurch gekennzeichnet, dass** die Prothese (2) eine Endoprothese ist.

13. Prothesensystem (1) nach einem der Ansprüche 1 bis 11, **dadurch gekennzeichnet, dass** die Prothese eine Exoprothese ist, wobei der primäre elektromechanische Wandler (3) und der elektromagnetische Wandler (4) an der Grenzfläche zum Gliedmaßenstumpf angeordnet sind und der Formkörper (7) im Stumpf implantiert angeordnet ist.

14. Prothesensystem (1) nach einem der vorhergehenden Ansprüche, **dadurch gekennzeichnet, dass** der Formkörper (7) aus einem keramischen oder kunststoffbasierten Material gefertigt ist.

## Claims

1. A prosthetic system (1) consisting of a prosthesis (2) and an encased electromagnetic transducer coil (6) with coil ends (8), which is spatially separated from the prosthesis (2) and acts as a bioelectric transducer, wherein a primary electromechanical transducer (3) with a directly coupled primary electromagnetic transducer (4) for the conversion of mechanical energy into electromagnetic waves (5), caused by movement, force, pressure and load changes acting on or in the prosthesis (2), is arranged on or in the prosthesis (2),
**characterized in that**
the coil ends (8), spatially separated therefrom, of the secondary electromagnetic transducer coil (6) converting the electromagnetic waves (5) into electrical signals are arranged as electrodes in a ceramic or plastic-based moulding (7) which is electrically nonconductive.

2. The prosthetic system (1) according to claim 1, **characterized in that** the primary electromechanical transducer (3) is a piezoelectric element, wherein the piezoelectric element responding to changes in movement and load generates electrical voltage signals which the coupled primary electromagnetic transducer (4) converts into a primary alternating electromagnetic field.

3. The prosthetic system (1) according to claim 1, **characterized in that** the primary electromechanical transducer (3) and the primary electromagnetic transducer (4) are united in one structural component or spatially separated.

4. The prosthetic system (1) according to claim 1, **characterized in that** the moulding (7) is open-pored and the pores of the moulding (7) are provided with active or electrically activatable signal substances and/or a biological matrix which, after implantation of the moulding (7), stimulate independent ingrowth of neuronal tissue into the moulding (7) so that a permanent signal interface is formed between neuronal tissue and the secondary electromagnetic transducer coil (6).

5. The prosthetic system (1) according to claims 1 to 4, **characterized in that** the moulding (7) is arranged spatially separate from the secondary electromagnetic transducer coil (6) or as a component of the secondary electromagnetic transducer coil (6).

6. The prosthetic system (1) according to claim 1, **characterized in that** the secondary electromagnetic transducer coil (6) is coupled to an amplifier module as a trigger.

7. The prosthetic system (1) according to claim 1, **characterized in that** the secondary electromagnetic transducer coil (6) comprises an integrated AC/DC converter.

8. The prosthetic system (1) according to claim 1, **characterized in that** the secondary electromagnetic transducer coil (6) is arranged in communication with an energy storage device and a signal generator which generates from an irregular biofeedback signal an always similar signal as a biofeedback signal which differs only in frequency or amplitude.

9. The prosthetic system (1) according to any one of the preceding claims for implantation in a human body, **characterized in that**, after implantation of the prosthetic system (1) has been performed, the ceramically encased secondary electromagnetic transducer coil (6) is positioned within the range of the primary electromagnetic waves (5) generated by the primary electromagnetic transducer (4) and the moulding (7) is positioned in close proximity to a nerve (9).

10. The prosthetic system (1) according to claim 9, **characterized in that** the bioelectrical feedback signals provided by the secondary electromagnetic transducer coil (6) via the coil ends (8) are available again as bioelectrical neuronal feedback from the prosthesis (2) for the vegetative system.

11. The prosthetic system according to any one of the preceding claims, **characterized in that** the electromagnetic transducer coil (6) is ceramically encased.

12. The prosthesis system (1) according to any one of the preceding claims, **characterized in that** the prosthesis (2) is an endoprosthesis.

13. The prosthetic system (1) according to any one of claims 1 to 11, **characterized in that** the prosthesis is an exoprosthesis, wherein the primary electromechanical transducer (3) and the electromagnetic transducer (4) are arranged at the interface with the limb stump and the moulding (7) is arranged in a manner implanted in the stump.

14. The prosthetic system (1) according to any one of the preceding claims, **characterized in that** the moulding (7) is made of a ceramic or plastic-based material.

## Revendications

1. Système de prothèse (1), constitué d'une prothèse (2) et d'une bobine de transducteur électromagnétique (6) encapsulée, spatialement séparée de la prothèse (2) et agissant comme transducteur bioélectrique, avec des extrémités de bobine (8), un transducteur électromécanique primaire (3) étant disposé, sur ou dans la prothèse (2), avec un transducteur électromagnétique primaire (4) directement couplé pour la conversion d'énergie mécanique en ondes électromagnétiques (5), provoquée par des variations de mouvement, de force, de pression et de charge agissant sur ou dans la prothèse (2),
**caractérisé en ce que**
les extrémités (8) de la bobine de transducteur électromagnétique secondaire (6), qui en sont spatialement séparées et qui convertissent les ondes électromagnétiques (5) en signaux électriques, sont disposées sous forme d'électrodes dans un corps moulé (7) en céramique ou à base de matière plastique, qui n'est pas conducteur de l'électricité.

2. Système de prothèse (1) selon la revendication 1, **caractérisé en ce que** le transducteur électromécanique primaire (3) est un élément piézoélectrique, l'élément piézoélectrique réagissant aux variations de mouvement et de charge générant des signaux de tension électrique que le transducteur électromagnétique primaire (4) couplé convertit en un champ électromagnétique primaire alternatif.

3. Système de prothèse (1) selon la revendication 1, **caractérisé en ce que** le transducteur électromécanique primaire (3) et le transducteur électromagnétique primaire (4) sont réunis dans un composant de construction ou séparés dans l'espace.

4. Système de prothèse (1) selon la revendication 1, **caractérisé en ce que** le corps moulé (7) est réalisé à pores ouverts et les pores du corps moulé (7) sont pourvus de substances de signalisation actives ou activables électriquement et/ou d'une matrice biologique qui, après implantation du corps moulé (7), stimulent une croissance autonome de tissu neuronal dans le corps moulé (7), de sorte qu'une interface de signalisation durable est formée entre le tissu neuronal et la bobine de transducteur électromagnétique secondaire (6).

5. Système de prothèse (1) selon les revendications 1 à 4, **caractérisé en ce que** le corps moulé (7) est spatialement séparé de la bobine de transducteur électromagnétique secondaire (6) ou est disposé en tant que partie constituante de la bobine de transducteur électromagnétique secondaire (6).

6. Système de prothèse (1) selon la revendication 1, **caractérisé en ce que** la bobine de transducteur électromagnétique secondaire (6) est couplée à un module amplificateur en tant que déclencheur.

7. Système de prothèse (1) selon la revendication 1, **caractérisé en ce que** la bobine de transducteur électromagnétique secondaire (6) comprend un convertisseur alternatif/continu intégré.

8. Système de prothèse (1) selon la revendication 1, **caractérisé en ce que** la bobine de transducteur électromagnétique secondaire (6) est disposée en liaison avec un accumulateur d'énergie et un générateur de signaux qui génère, à partir d'un signal de rétroaction biologique irrégulier, un signal toujours identique en tant que signal de rétroaction biologique, qui ne diffère que par la fréquence ou l'amplitude.

9. Système de prothèse (1) selon l'une quelconque des revendications précédentes, destiné à être implanté dans un corps humain, **caractérisé en ce qu'**une fois l'implantation du système de prothèse (1) réalisée, la bobine de transducteur électromagnétique secondaire (6) encapsulée dans de la céramique est positionnée à portée des ondes électromagnétiques primaires (5) générées par le transducteur électromagnétique primaire (4) et le corps moulé (7) est positionné à proximité immédiate d'un nerf (9).

10. Système de prothèse (1) selon la revendication 9, **caractérisé en ce que** les signaux de rétroaction bioélectriques fournis par la bobine de transducteur électromagnétique secondaire (6) par l'intermédiaire des extrémités de bobine (8) sont à nouveau disponibles pour le système végétatif en tant que rétroaction bioélectrique-neuronale provenant de la prothèse (2).

11. Système de prothèse (1) selon l'une quelconque des revendications précédentes, **caractérisé en ce que** la bobine de transducteur électromagnétique (6) est encapsulée dans de la céramique.

12. Système de prothèse (1) selon l'une quelconque des revendications précédentes, **caractérisé en ce que** la prothèse (2) est une endoprothèse.

13. Système de prothèse (1) selon l'une quelconque des revendications 1 à 11, **caractérisé en ce que** la prothèse est une exoprothèse, le transducteur électromécanique primaire (3) et le transducteur électromagnétique (4) étant disposés à l'interface avec le moignon de membre et le corps moulé (7) étant disposé de manière implantée dans le moignon.

14. Système de prothèse (1) selon l'une quelconque des revendications précédentes, **caractérisé en ce que** le corps moulé (7) est fabriqué à partir d'un matériau céramique ou à base de matière plastique.
